# EUROPEAN PATENT APPLICATION

(11) **EP 4 119 193 A1**
(43) Date of publication of application: **18.01.2023**
(21) Application number: 20919583.3
(22) Date of filing: 29.10.2020
(51) Int. Cl.: A61P 13/00, A61P 43/00, C12N 5/071, C12N 5/074, A61K 35/36, A61K 35/37, A61L 27/36, A61L 27/38

(54) **METHOD FOR CULTURING CELLS DERIVED FROM EPITHELIAL TISSUE, AND COMPOSITION CONTAINING CELLS CULTURED BY SAID CULTURE METHOD**

(30) Priority: 21.02.2020 JP 2020028226
(71) Applicant: JBM Incorporation, Tokyo 133-0052 (JP); GN Corporation Co Ltd, Kofu-shi, Yamanashi 400-0866 (JP)
(72) Inventor: KATOH, Shojiro, Tokyo 133-0052 (JP); ABRAHAM, Samuel JK, Kofu-shi, Yamanashi 400-0866 (JP)
(74) Representative: Forstmeyer, Dietmar
(86) International application number: PCT/JP2020/040590
(87) International publication number: WO 2021/166330

(57) **Abstract**

The purpose of the present invention is to provide: a method for maintaining or enhancing the activity of a cell mass separated from an epithelial tissue; a method for increasing the proliferation ability of cells in an epithelial tissue; a method for producing a cell mass employing these methods; a pharmaceutical composition containing the cell mass; and a method for treating a disease using the cell mass. The purpose can be solved by culturing a cell mass separated from an epithelial tissue or an epithelial tissue with a thermoreversible polymer.

## Description

### [Technical Field]

The present invention relates to a method for maintaining or enhancing the activity of a cell population separated from epithelial tissue, a method for enhancing the proliferating ability of cells in epithelial tissue, a method for producing a cell population using these methods, a pharmaceutical composition containing the cell population, and a method for treating a disease using the cell population, and the like.

### [Background Art]

In recent years, cell therapy has begun to be performed for repairing damaged tissues and the like. In order to perform cell therapy, cells need to engraft in the transplanted tissue and perform desired functions. However, especially when cells separated from an epithelial tissue are used for cell therapy, it is known that traits of the cells which have been retained when the cells have maintained the epithelial tissue are reduced or disappeared through culturing (Patent Document 1).

### [Citation List]

### Non-Patent Document

[Non-Patent Document 1] Follmann et al., Toxicol In Vitro. 2000 Oct; 14 (5): 435-45.

### [Summary of Invention]

### [Problems to Be Solved by Invention]

The present invention aims to provide a method for maintaining or enhancing the activity of a cell population separated from epithelial tissue, a method for enhancing the proliferating ability of cells in epithelial tissue, a method for producing a cell population using these methods, a pharmaceutical composition containing the cell population, and a method for treating a disease using the cell population.

### [Means of Solving Problems]

The present inventors have conducted diligent research to solve the above problems, and found that activities such as epithelial maintenance ability, tissue engraftment ability and epithelial tissue regeneration ability of a cell population can be enhanced by culturing the cell population separated from an epithelial tissue in a thermoreversible gelation polymer, and that the proliferating ability of cells in the epithelial tissue, in particular the proliferating ability of epithelial stem cells can be enhanced by culturing the epithelial tissue as it is in a thermoreversible gelation polymer.

Furthermore, the present inventors have found that the cell population cultured in plate culture and the cell population cultured in a thermoreversible gelation polymer have different properties, and that damaged epithelial tissues can be repaired using a mixture of both cell populations; and the present inventors have completed the present invention.

That is, the present invention relates to the following.
(1) A method for maintaining or enhancing an activity selected from the group consisting of epithelial maintenance ability, tissue engraftment ability and epithelial tissue regeneration ability of a cell population separated from an epithelial tissue, which comprises a step of culturing the cell population in a thermoreversible gelation polymer.
(2) The method according to (1) above, wherein the activity selected from the group consisting of epithelial maintenance ability, tissue engraftment ability and epithelial tissue regeneration ability is to maintain or enhance the expression of one or more proteins selected from the group consisting of cytokeratin, EGFR, p63, p75 and integrin β or genes encoding said proteins.
(3) A method for enhancing the proliferating ability of cells in an epithelial tissue, which comprises a step of culturing the epithelial tissue in a thermoreversible gelation polymer.
(4) The method according to (3) above, wherein the cells are epithelial stem cells.
(5) The method according to (3) or (4) above, which comprises a step of preserving the epithelial tissue in a thermoreversible gelation polymer before the step of culturing said tissue in the thermoreversible gelation polymer.
(6) The method according to any one of (1) to (5) above, wherein the epithelial tissue is derived from the oral mucosa.
(7) The method according to any one of (1) to (6) above, wherein the thermoreversible gelation polymer is composed by binding a plurality of blocks having a cloud point selected from the group consisting of polypropylene oxide, copolymers of propylene oxide and other alkylene oxides, poly N-substituted acrylamide derivatives, poly N-substituted methacrylamide derivatives, copolymers of N-substituted acrylamide derivatives and N-substituted methacrylamide derivatives, polyvinyl methyl ether, and partially acetylated polyvinyl alcohol, and a hydrophilic block.
(8) The method according to any one of (1) to (7), wherein the thermoreversible gelation polymer is composed by binding poly-N-isopropylacrylamide and polyethylene oxide.
(9) A method for producing a cell population, comprising the method according to any one of (1) to (8).
(10) A cell population obtained by the method according to any one of (1) to (8) or the production method according to (9) .
(11) A method for producing an epithelial tissue, comprising the method according to any one of (3) to (8).
(12) The epithelial tissue obtained by the production method according to (11).
(13) A pharmaceutical composition comprising the cell population according to (10) or the epithelial tissue according to (12).
(14) The pharmaceutical composition according to (13) for application to a defect in a lumen.
(15) The pharmaceutical composition according to (14), wherein the lumen is the urethra or the ureter.

Furthermore, the present invention relates to the following.
[1] A composition for repairing damage to an epithelial tissue, comprising:
   (1) cells derived from the same or different epithelial tissue as the epithelial tissue to be repaired, which have been cultured in plate culture, and
   (2) cells derived from the same or different epithelial tissue as the epithelial tissue to be repaired, which have been cultured in a thermoreversible gelation polymer.
[2] The composition according to [1] above, wherein the cells cultured in plate culture produce insulin-like growth factor 1 (IGF-1).
[3] The composition according to [1] or [2] above, wherein the cells cultured in a thermoreversible gelation polymer are positive for epithelial cell markers AE1 and AE3.
[4] The composition according to any one of [1] to [3] above, wherein the epithelial tissue to be repaired include epithelial tissues of oral cavity, nasal cavity, trachea, alveoli, vascular endothelium, lymphatic endothelium, bile duct, urinary tract, sweat gland, cornea, esophagus, stomach, mammary duct, endometrial membrane, cervix of uterus, large intestine, colon, kidney or bladder.
[5] The composition according to any one of [1] to [4] above, wherein the cells cultured in plate culture and the cells cultured in a thermoreversible gelation polymer are cells derived from the same epithelial tissue.
[6] The composition according to [5] above, wherein the cells cultured in plate culture and the cells cultured in a thermoreversible gelation polymer are derived from the oral mucosa.
[7] The composition according to any one of [1] to [6] above, wherein the thermoreversible gelation polymer is composed by binding a plurality of blocks having a cloud point selected from the group consisting of polypropylene oxide, copolymers of propylene oxide and other alkylene oxides, poly N-substituted acrylamide derivatives, poly N-substituted methacrylamide derivatives, copolymers of N-substituted acrylamide derivatives and N-substituted methacrylamide derivatives, polyvinyl methyl ether, and partially acetylated polyvinyl alcohol, and a hydrophilic block.
[8] The composition according to any one of [1] to [7] above, wherein the thermoreversible gelation polymer is composed by binding poly-N-isopropylacrylamide and polyethylene oxide.

### Advantageous Effects of Invention

In the cell population produced by the method of the present invention, cytokeratin, EGFR (epidermal growth factor receptor), p63 and/or integrin β are highly expressed, and the cell population is excellent in engraftment and regeneration in a tissue and in subsequent survivability and functionality, as well as sustainability of functions; therefore it can achieve healthy repair of epithelial tissue. In particular, the composition of the present invention can repair and/or regenerate and cure damaged or dysfunctional epithelium by using the mixture of a cell population matured as epithelial cells by culturing in a thermoreversible gelation polymer and a cell population which produces growth factors such as insulin-like growth factor 1 (IGF-1) by plate culture. In addition, the epithelial tissue produced by the method of the present invention has high proliferating ability of cells, and in particular, a large number of epithelial stem cells can be obtained; therefore, by separating such cells, a high-quality cell population that can be used for repairing epithelial tissue can be obtained. Furthermore, since each step in the present invention requires a little labor and cost, the production method of the present invention can be widely utilized for producing a cell population separated from an epithelial tissue.

### [Brief Description of Drawings]

[Fig. 1] Figure 1 shows photographs of cell populations cultured in plate culture or in thermoreversible gelation polymer, immunostained with an anti-AE1/AE3 antibody.
[Fig. 2] Figure 2 shows photographs of cell populations cultured in plate culture or in thermoreversible gelation polymer, immunostained with an anti-EGFR antibody.
[Fig. 3] Figure 3 shows photographs of cell populations cultured in plate culture or in thermoreversible gelation polymer, immunostained with an anti-integrin β antibody.
[Fig. 4] Figure 4 shows photographs of cell populations cultured in plate culture or in thermoreversible gelation polymer, immunostained with an anti-P75 antibody.
[Fig. 5] Figure 5 shows proliferativeness of epithelial tissues that are transported in thermoreversible gelation polymer (sample A) or ECS (sample B), then cultured in plate culture (DMEM or Cnt-PR) or in thermoreversible gelation polymer.
[Fig. 6] Figure 6 shows the results of flow cytometry of each cell at day 0 of collection (left end), at day 7, day 14, and day 21 of plate culture (2D) (upper right), and at day 7, day 14, and day 21 of TGP culture (3D) (lower right).
[Fig. 7] Figure 7 shows the analysis results of epithelial cell markers AE1 and AE3 in the cells cultured in plate culture (2D) (left) and the cells cultured in TGP (3D) (right).
[Fig. 8] Figure 8 shows the analysis results of epithelial cell marker CK3 by RT-PCR in the cells cultured in plate culture (2D) and the cells cultured in TGP (3D).
[Fig. 9] Figure 9 shows the results of cell transplantation experiment in the ureteral epithelium of an animal using cells derived from buccal tissue.

### [Embodiments for Carrying out Invention]

Unless defined otherwise herein, all technical and scientific terms used herein have the same meaning as those commonly understood by one of ordinary skill in the art. All patents, applications, published applications and other publications referenced herein are hereby incorporated by reference in their entirety.

### <Method of increasing activity of cell population separated from epithelial tissue>

The present invention is a method for maintaining or enhancing the activity selected from the group consisting of the epithelial maintenance ability, tissue engraftment ability and epithelial tissue regeneration ability of a cell population separated from an epithelial tissue, which comprises a step of culturing the cell population in a thermoreversible gelation polymer.

"Epithelial tissue" in the present disclosure refers to a tissue derived from a living body comprising at least one or both of epithelial cells and epithelial stem cells. The epithelial tissue in the present disclosure may comprise, without limitation, one or more epithelia selected from the group consisting of simple squamous epithelium, simple cubic epithelium, simple columnar epithelium, stratified squamous epithelium, stratified cubic epithelium, and stratified columnar epithelium. The epithelial tissue of the present invention preferably further comprises a basement membrane. The epithelial tissue in the present disclosure may be, but is not limited to, an epithelial tissue obtained from oral cavity, nasal cavity, trachea, alveoli, vascular endothelium, lymphatic endothelium, bile duct, urinary tract such as tubule, sweat gland, cornea, esophagus, stomach, mammary duct, endometrial membrane, cervix of uterus, large intestine, colon, kidney or bladder. The epithelial tissue in the present disclosure may be in the form of being bound to one or more tissues selected from the group consisting of connective tissue, muscle tissue and nervous tissue. Typically, the epithelial tissue may be in the form of being connected to connective tissue via the basement membrane.

In one embodiment, the epithelial tissue of the present invention may be in the form of a mucous membrane. The mucous membrane may be in the form of comprising mucosal epithelium, lamina propria of mucous membrane and/or lamina muscularis mucosae. In one embodiment, the mucous membrane may be, but is not limited to, a mucous membrane selected from oral mucosa, nasal mucosa, and gastrointestinal mucosa. In one embodiment, oral mucosa is preferred. The oral mucosa is the mucous membrane lining the inside of the mouth and is composed of stratified squamous epithelium and lamina propria of mucous membrane. In one embodiment of the present invention, the oral mucosa may be an oral mucosa selected from masticatory mucosa, lining mucosa and specialized mucosa, and is preferably lining mucosa. In one embodiment of the present invention, the lining mucosa may be one or more lining mucosa selected from buccal mucosa, labial mucosa and alveolar mucosa. From the viewpoint of proliferative power, buccal mucosa is preferable. The buccal mucosa means the inside lining of the cheeks.

In one embodiment, the buccal mucosa comprises epithelial cells and/or epithelial stem cells. In another embodiment, in addition to epithelial cells and/or epithelial stem cells, the buccal mucosa further comprises fibroblasts, adipocytes, melanosites, lymphocytes, Langerhans cells and/or neutrophils.

The method for collecting an epithelial tissue may be a usual method used for collecting biological tissue in the art, and the tissue can be collected from a subject using a scalpel, tweezers, a biopsy punch, etc., without limitation. In one embodiment of the present invention, the epithelial tissue may be a damaged epithelial tissue or an epithelial tissue with reduced number of viable cells due to storage after collection. The epithelial tissue in the present invention can be derived from any organism. Such organisms include, but are not limited to, for example, humans, non-human primates, dogs, cats, pigs, horses, goats, sheep, rodents (e.g., mice, rats, hamsters, guinea pigs, etc.), rabbits and the like.

The "cell population separated from epithelial tissue" in the method of the present invention means a cell population in which there are the same or different types of cells obtained by separation treatment of the epithelial tissue (for example, enzymatic treatment, shredding treatment), and/or epithelial tissue pieces remaining after separation treatment. The epithelial tissue to be subjected to the separation treatment may be, but is not limited to, an epithelial tissue immediately after being collected, or an epithelial tissue cultured in a thermoreversible gelation polymer as described later. The cell population comprises either one or both of epithelial cells and/or epithelial stem cells. In another embodiment, the cell population may further comprise fibroblasts, adipocytes, melanosites, lymphocytes, Langerhans cells and/or neutrophils in addition to epithelial cells and/or epithelial stem cells. Another example of "epithelial tissue pieces remaining after separation treatment" is the epithelial tissue that cannot be completely separated by a separation treatment. In one embodiment of the present invention, the cell population separated from the epithelial tissue consists of the same or different types of cells obtained by the separation treatment of the epithelial tissue. In one embodiment of the present invention, the cell population separated from the epithelial tissue comprises a piece of epithelial tissue that remains after the separation treatment. Since the epithelial tissue piece comprises a large amount of epithelial stem cells, which enhances the epithelial tissue regeneration ability of the cell population, it is preferable that the cell population comprises epithelial tissue pieces remaining after the separation treatment.

As the separation treatment of epithelial tissue, a method usually used as a separation treatment of biological tissue in the art, for example, an enzymatic method or a physical method can be used. The enzymatic method is, but not limited to, the use of one or more enzymes for incubation at a temperature at which cells constituting the epithelial tissue are viable (for example, 37°C). In the separation treatment by an enzymatic method, an enzyme commonly used in the art in separating cells from a tissue can be used under treatment conditions of corresponding temperature, concentration and time. Enzymes are not limited as long as epithelial tissue can be separated, and examples thereof include enzymes such as Dispase I or II, collagenase, metalloprotease, trypsin, hyaluronidase, pepsin, aminopeptidase, lipase, amylase, or a recombinant thereof, and a mixture thereof. From the viewpoint of degrading epithelial tissue in a short time and with minimal invasiveness, as an enzyme, preferably any one of Dispase I or II, collagenase, metalloprotease, accutase or trypsin may be used alone, or two or more kinds may be used in combination. From the viewpoint that a cell population that favorably expresses cytokeratin, EGFR, p63, p75 or integrin β can be selected by a separation treatment with moderate invasiveness, Dispase I and trypsin are preferably used in combination.

When Dispase I is used, it may be, without limitation, 100 PU/mL to 10000 PU/mL, 150 to 5000 PU/mL; and from the viewpoint of improving the expression of cytokeratin, EGFR, p63, p75 or integrin β in the separated cell population, it is preferably 190 to 2000 PU/mL, more preferably 200 to 1000 PU/mL, and particularly preferably 1000 PU/mL. The separation treatment time of epithelial tissue is not limited as long as viable cells are not killed, and the time may be, for example, 5 minutes to 2 hours, 10 minutes to 1.5 hours, 15 minutes to 2 hours, 20 to 90 minutes. It is preferably 60 minutes from the viewpoint of improving the expression of cytokeratin, EGFR, p63, p75 and integrin β in the separated cell population. In the case of trypsin, without limitation, in the form of trypsin-EDTA it may be 0.05% to 2.5%, 0.1% solution to 1%, 0.15% solution to 0.3%, and more preferably 0.2 to 0.25%; from the viewpoint of improving the expression of cytokeratin, EGFR, p63, p75 and integrin β in the separated cell population, it is preferably 0.25%. The separation treatment time with trypsin-EDTA is not limited, and may be 1 minute to 2 hours, 3 minutes to 1 hour, 10 minutes to 40 minutes; from the viewpoint of maintaining the traits of epithelial cells of the separated cell population, it is preferably 15 minutes. When Dispase and trypsin-EDTA are used in combination, each concentration and treatment time can be arbitrarily combined without limitation. From the viewpoint of maintaining the traits of epithelial cells and degrading epithelial stem cells with minimal invasiveness, a combination of treatment with Dispase I (1000 PU/mL) at 37°C for 60 minutes and treatment with trypsin-EDTA (0.25% solution) at 37°C for 15 minutes is preferred.

Examples of the physical method include, but are not limited to, a method of shredding or crushing epithelial tissue using a scalpel, ultrasonic waves, a homogenizer, a strainer, or the like. The physical method can be optionally combined with the enzymatic method detailed above. From the viewpoint of maintaining the traits of epithelial cells and degrading epithelial stem cells with minimal invasiveness, as an example of such combination without limitation, it is preferable to treat the tissue with Dispase I (1000 PU/mL) at 37°C for 60 minutes and shred it, then treat the shredded tissue pieces with trypsin-EDTA (0.25% solution) at 37°C for 15 minutes.

The separated cells may be differentiated as: the cells separated by the separation treatment using any known method such as a filter and a cell strainer; and the epithelial tissue pieces remaining without being separated. By the differentiation, cell populations with a uniform size or function can be obtained. The remaining unseparated epithelial tissue pieces contain a large amount of epithelial stem cells, and this enhances the tissue regeneration ability of the cell population; therefore, it is preferable that the cells separated by the separation treatment and the remaining unseparated epithelial tissue pieces are present in mixture without the differentiation.

### (Thermoreversible gelation polymer)

The thermoreversible gelation polymer in the method of the present invention (also referred to as "TGP" in the present specification) refers to a polymer which forms a crosslinked structure or a network structure in a thermally reversible manner, and based on said structure, which has a property of being able to thermally reversibly form a hydrogel that holds in its inside a separation liquid such as water. In addition, the hydrogel refers to a gel comprising a crosslinked or network structure made of polymers, and water that is supported or retained in said structure. In the present invention, since the thermoreversible gelation polymer forms an environment similar to the epithelial tissue in the living body by the crosslinked structure or network structure peculiar to the thermoreversible gelation polymer, any thermoreversible gelation polymers can be used in the method of the present invention.

### (Sol-gel transition temperature)

Definition and measurement of "sol state", "gel state" and "sol-gel transition temperature" in the present invention are based on the definitions and measurements described in the literature (H. Yoshioka et al., Journal of Macromolecular Science, A31(1), 113 (1994)). That is, the dynamic elastic modulus of a sample at an observation frequency of 1 Hz is measured by gradually changing the temperature (1°C/1 minute) from low temperature side to high temperature side, and the temperature at the point where the storage elastic modulus of the sample (G', elastic term) exceeds the loss elastic modulus (G", viscosity term) is defined as the sol-gel transition temperature. Generally, a state with G">G' is defined as a sol and a state with G"<G' is defined as a gel. In measuring the sol-gel transition temperature, the following measurement conditions can be preferably used.

### <Measurement conditions for dynamic/loss elastic modulus>

Measuring device (trade name): Stress control type rheometer AR500, manufactured by TA Instruments
Concentration of sample solution (or separation liquid) (as the concentration of "hydrogel-forming polymer having sol-gel transition temperature"): 10 (weight)%
Amount of sample solution: Approximately 0.8 g
Shape/dimensions of measurement cell: Acrylic parallel disk (diameter 4.0 cm), gap 600 µm
Measurement frequency: 1 Hz
Applied stress: Within linear region.

In the present invention, the above sol-gel transition temperature is preferably higher than 0°C and 37°C or lower, and furthermore preferably it is higher than 5°C and 35°C or lower (in particular 10°C or higher and 33°C or lower). A TGP having such a suitable sol-gel transition temperature can be easily selected from specific compounds as described later according to the above-mentioned screening method (sol-gel transition temperature measurement method).

The TGP of the present invention is not particularly limited as long as it exhibits a thermoreversible sol-gel transition as described above (that is, has a sol-gel transition temperature). As specific examples of the polymer wherein its aqueous solution has a sol-gel transition temperature and reversibly exhibits a sol state at a temperature lower than the transition temperature, the following are known: for example, polyalkylene oxide block copolymers typified by a block copolymer of polypropylene oxide and polyethylene oxide, etc.; etherified cellulose such as methyl cellulose and hydroxypropyl cellulose, etc.; chitosan derivatives (K. R. Holme, et al. Macromolecules, 24, 3828 (1991)) and the like.

### (Suitable hydrogel-forming polymer)

The hydrogel-forming polymer utilizing a hydrophobic bond for crosslink formation, which can be suitably used as a TGP of the present invention, is preferably composed by binding a plurality of blocks having a cloud point and a hydrophilic block, from the viewpoint of stably maintaining the surroundings of the cell population or epithelial tissue without separating the TGP from the medium.

The hydrophilic block is preferably present in order for the hydrogel to become water soluble at a temperature lower than the sol-gel transition temperature, and the plurality of blocks having a cloud point are preferably present in order for the hydrogel to change to a gel state at a temperature higher than the sol-gel transition temperature.

In other words, a block with a cloud point dissolves in water at a temperature lower than the cloud point and becomes insoluble in water at a temperature higher than the cloud point, so that the block serves as a cross-linking point consisting of hydrophobic bonds for the formation of a gel at the temperature higher than the cloud point. That is, the cloud point derived from the hydrophobic bond corresponds to the sol-gel transition temperature of the hydrogel.

However, the cloud point and the sol-gel transition temperature do not necessarily have to match. This is because the cloud point of the above-mentioned "block having a cloud point" is generally affected by the binding between said block and a hydrophilic block.

The hydrogel used in the present invention utilizes the property that the hydrophobic bond not only becomes stronger with increasing temperature but also the change is reversible with respect to temperature. It is preferable for the TGP to have a plurality of "blocks having a cloud point", from the viewpoint that multiple cross-linking points are formed in one molecule and a gel with excellent stability is formed, and that a cell population or epithelial tissue can grow stably against reversible temperature changes.

In contrast, as described above, the hydrophilic block in the TGP has a function of changing the TGP to be water-soluble at a temperature lower than the sol-gel transition temperature, and has a function of forming a state of a hydrogel, while preventing the hydrogel from coagulating and settling due to excessive increase in a hydrophobic binding force at a temperature higher than the transition temperature.

Furthermore, it is desirable that the TGP used in the present invention is those that can be degraded and absorbed in vivo, from the viewpoint of using the cell population or epithelial tissue of the present invention in cell therapy. That is, it is preferable that the TGP of the present invention is degraded in a living body by a hydrolysis reaction or an enzymatic reaction to become a low molecular weight substance harmless to the living body, and is absorbed and excreted.

When the TGP of the present invention is the one that is composed by binding a plurality of blocks having a cloud point and a hydrophilic block, it is preferable that at least either one of the block having a cloud point and the hydrophilic block, preferably both of them, is/are degraded and absorbed in vivo.

### (A plurality of blocks having a cloud point)

The block having a cloud point is preferably a block of a polymer having a negative solubility -temperature coefficient in water, and more specifically, a polymer selected from the group consisting of polypropylene oxide, copolymers of propylene oxide and other alkylene oxides, poly N-substituted acrylamide derivatives, poly N-substituted methacrylamide derivatives, copolymers of N-substituted acrylamide derivatives and N-substituted methacrylamide derivatives, polyvinyl methyl ether, and partially acetylated polyvinyl alcohol can be preferably used. From the viewpoint of stably culturing the cell population or epithelial tissue of the present invention, a poly N-substituted acrylamide derivative is preferable.

In order to make the block having a cloud point those that can be degraded and absorbed in a living body, it is effective to make it a polypeptide consisting of a hydrophobic amino acid and a hydrophilic amino acid. Alternatively, a polyester-type biodegradable polymer such as polylactic acid or polyglycolic acid may be used as a block having a cloud point that is degraded and absorbed in a living body.

The fact that the cloud point of the above polymer (block having a cloud point) is higher than 4°C and 40°C or less is preferable from the viewpoint of making the sol-gel transition temperature of the polymer (a compound in which a plurality of blocks having a cloud point and a hydrophilic block are bonded) used in the present invention higher than 0°C and 37°C or lower.

Here, the cloud point may be measured as follows: for example, an aqueous solution of about 1 mass% of the above polymer (block having a cloud point) is cooled to obtain a transparent uniform solution, and the temperature is gradually increased (heating rate of about 1°C/min); then the temperature at which the solution becomes cloudy for the first time is set as the cloud point.

Specific examples of poly N-substituted acrylamide derivatives and poly N-substituted methacrylamide derivatives that can be used in the present invention are listed below.

Poly-N-acroylpiperidine; poly-N-n-propylmethacrylamide; poly-N-isopropylacrylamide; poly-N, N-diethylacrylamide; poly-N-isopropylmethacrylamide; poly-N-cyclopropylacrylamide; poly-N-acryloyl-pyrrolidine; poly-N,N-ethylmethylacrylamide; poly-N-cyclopropylmethacrylamide; poly-N-ethylacrylamide. From the viewpoint of stably culturing the cell population or epithelial tissue of the present invention, poly-N-isopropylacrylamide is preferable.

The above polymer may be a homopolymer, or a copolymer of a monomer constituting the above polymer and other monomer. As the other monomer constituting such a copolymer, either a hydrophilic monomer or a hydrophobic monomer can be used. In general, copolymerization with a hydrophilic monomer raises the cloud point of the product, and copolymerization with a hydrophobic monomer lowers the cloud point of the product. Therefore, by selecting these monomers to be copolymerized, it is possible to obtain a polymer having a desired cloud point (for example, a cloud point higher than 4°C and 40°C or lower).

### (Hydrophilic monomer)

Examples of the above hydrophilic monomer include N-vinylpyrrolidone, vinylpyridine, acrylamide, methacrylamide, N-methylacrylamide, hydroxyethyl methacrylate, hydroxyethyl acrylate, hydroxymethyl methacrylate, hydroxymethyl acrylate, acrylic acids and methacrylic acids having an acidic group and salts thereof, vinyl sulfonic acid, styrene sulfonic acid, etc., as well as N,N-dimethylaminoethyl methacrylate, N,N-diethylaminoethyl methacrylate, and N,N-dimethylaminopropyl acrylamide having a basic group and salts thereof, but are not limited thereto.

### (Hydrophobic monomer)

Meanwhile, examples of the above hydrophobic monomer include acrylate derivatives and methacrylate derivatives such as ethyl acrylate, methyl methacrylate and glycidyl methacrylate, etc., N-substituted alkyl methacrylamide derivatives such as N-n-butyl methacrylamide, etc., vinyl chloride, acrylonitrile, styrene, vinyl acetate, etc., but are not limited thereto.

### (Hydrophilic block)

Meanwhile, specific examples of the hydrophilic block to be bound to the above-mentioned block having a cloud point include methylcellulose, dextran, polyethylene oxide, polyvinyl alcohol, poly N-vinylpyrrolidone, polyvinylpyridine, polyacrylamide, polymethacrylamide, poly N-methylacrylamide, polyhydroxymethyl acrylate, polyacrylic acid, polymethacrylic acid, polyvinylsulfonic acid, polystyrene sulfonic acid and salts thereof; poly N,N-dimethylaminoethyl methacrylate, poly N,N-diethylaminoethyl methacrylate, poly N,N-dimethylaminopropyl acrylamide, and salts thereof, etc. In one embodiment of the present invention, when poly-N-isopropylacrylamide is used in a thermoreversible gel as the plurality of blocks having a cloud point, an environment similar to the in vivo environment of epithelial tissue is formed, whereby the proliferating ability of the cell population or epithelial tissue of the present invention is increased; therefore, as the hydrophilic block that binds to the acrylamide, polyethylene oxide is preferred.

In addition, it is desirable that the hydrophilic block is degraded, metabolized and excreted in the living body; and hydrophilic biopolymers such as proteins, e.g., albumin or gelatin, and polysaccharides, e.g., hyaluronic acid, heparin, chitin or chitosan are preferably used.

The method for binding the block having a cloud point and the above hydrophilic block is not particularly limited; for example, this can be done by introducing a polymerizable functional group (for example, an acryloyl group) into either of the above blocks, and copolymerizing with a monomer giving the other block. In addition, a bound substance of the block having a cloud point and the above-mentioned hydrophilic block can also be obtained by block copolymerization of a monomer giving a block having a cloud point and a monomer giving a hydrophilic block. Furthermore, binding between the block having a cloud point and the hydrophilic block can be achieved by introducing a reactive functional group (for example, a hydroxyl group, an amino group, a carboxyl group, an isocyanate group, etc.) into the both blocks in advance, and binding the both blocks by a chemical reaction. At this time, a plurality of reactive functional groups are usually introduced into the hydrophilic block. In addition, binding between polypropylene oxide having a cloud point and a hydrophilic block is achieved by, for example, repeatedly and sequentially polymerizing the propylene oxide and the monomer constituting the "other hydrophilic block" (for example, polyethylene oxide) by anionic polymerization or cationic polymerization, to obtain a block copolymer in which polypropylene oxide and the "hydrophilic block" (for example, polyethylene oxide) are bonded. Such a block copolymer can also be obtained by introducing a polymerizable group (for example, an acryloyl group) at the end of polypropylene oxide and then copolymerizing with a monomer constituting the hydrophilic block. Furthermore, the polymer used in the present invention can also be obtained by introducing a functional group capable of binding reaction with a functional group (for example, a hydroxyl group) at the terminal of polypropylene oxide into a hydrophilic block and reacting the both of them. In addition, the TGP used in the present invention can also be obtained by connecting a material such as Pluronic F-127 (trade name, manufactured by Asahi Denka Kogyo Co., Ltd.) in which polyethylene glycol is bonded to both ends of polypropylene glycol.

The polymer of the present invention in the embodiment comprising the blocks having a cloud point is completely dissolved in water and shows a sol state at a temperature lower than the cloud point, because the above-mentioned "blocks having a cloud point" together with the hydrophilic block existing in the molecule are water-soluble at that temperature. However, when the temperature of the aqueous solution of this polymer is heated to a temperature higher than the above-mentioned cloud point, the "blocks having a cloud point" existing in the molecule become hydrophobic, and they are associated between different molecules due to hydrophobic interaction.

On the other hand, since the hydrophilic block is water-soluble even at this time (when heated to a temperature higher than the cloud point), the polymer of the present invention produces a hydrogel having a three-dimensional network structure in water with a site of hydrophobic association between the blocks having a cloud point as the cross-linking point. When the temperature of this hydrogel is cooled again to a temperature lower than the cloud point of the "blocks having a cloud point" existing in the molecule, the blocks having the cloud point become water-soluble, the cross-linking points due to the hydrophobic association are released, and the hydrogel structure disappears; and the TGP of the present invention becomes a complete aqueous solution again. Thus, the sol-gel transition of the polymer of the present invention in a preferred embodiment is based on reversible changes between hydrophilicity and hydrophobicity at the cloud point of the blocks having a cloud point existing in the molecule; therefore, the polymer of the present invention has complete reversibility in response to temperature changes.

According to studies by the present inventors, the above-mentioned delicate hydrophilic-hydrophobic balance of the TGP in water is considered to contribute to the stability of cells when they are stored in water at low temperature.

### (Solubility of gel)

As described above, the hydrogel-forming polymer of the present invention comprising at least a polymer having a sol-gel transition temperature in an aqueous solution shows substantial water insolubility at a temperature (d°C) higher than the sol-gel transition temperature, and reversibly shows water solubility at a temperature (e°C) lower than the sol-gel transition temperature.

The above-mentioned high temperature (d°C) is preferably 1°C or higher than the sol-gel transition temperature, and more preferably 2°C or higher (in particular 5°C or higher). In addition, the above-mentioned "substantial water insolubility" means that the amount of the above-mentioned polymer dissolved in 100 ml of water at the above-mentioned temperature (d°C) is preferably 5.0 g or less (furthermore, 0.5 g or less, and in particular 0.1 g or less).

Meanwhile, the above-mentioned low temperature (e°C) is preferably 1°C or more (absolute value) lower than the sol-gel transition temperature, and furthermore preferably 2°C or more lower (in particular 5°C or more lower). In addition, the above-mentioned "water solubility" means that the amount of the above-mentioned polymer dissolved in 100 ml of water at the above-mentioned temperature (e°C) is preferably 0.5 g or more (furthermore, 1.0 g or more). Furthermore, "reversibly shows water solubility" means that even after the above-mentioned TGP aqueous solution is once gelled (at a temperature higher than the sol-gel transition temperature), it shows the above-mentioned water solubility at a temperature lower than the sol-gel transition temperature.

It is preferable that the 10% aqueous solution of the above polymer exhibits a viscosity of 10 to 3,000 centipoise (furthermore, 50 to 1,000 centipoise) at 5°C. Such viscosity is preferably measured under the following measurement conditions, for example.
Viscometer: Stress control type rheometer (model name: AR500, manufactured by TA Instruments)
Rotor diameter: 60 mm
Rotor shape: Parallel flat plate

Even if the aqueous solution of the TGP of the present invention is gelled at a temperature higher than the above-mentioned sol-gel transition temperature and then immersed in a large amount of water, the gel does not substantially dissolve. The above-mentioned characteristics of the hydrogel formed by the above-mentioned TGP can be confirmed, for example, as follows.

That is, 0.15 g of the TGP is dissolved in 1.35 g of distilled water at a temperature lower than the above-mentioned sol-gel transition temperature (for example, under ice cooling) to prepare a 10 wt% aqueous solution, and the aqueous solution is injected in a plastic petri dish having a diameter of 35 mm and heated to 37°C, to obtain the formation of a gel having a thickness of about 1.5 mm in the petri dish; and then the weight (f gram) of the entire petri dish containing the gel is measured. Then, the entire petri dish containing the gel was allowed to stand in 250 ml of water at 37°C for 10 hours, and the weight (g gram) of the entire petri dish containing the gel was measured to evaluate the presence or absence of dissolution of the gel from the gel surface. At this time, in the hydrogel-forming polymer of the present invention, the weight loss rate of the above-mentioned gel, that is, (f-g)/f is preferably 5.0% or less, and furthermore preferably 1.0% or less (in particular 0.1% or less).

When the TGP aqueous solution of the present invention is gelled at a temperature higher than the above-mentioned sol-gel transition temperature, and then immersed in a large amount of water (about 0.1 to 100 times the gel in volume), the gel does not dissolve for a long period of time. Such properties of the polymer used in the present invention are achieved, for example, by the presence of two or more (a plurality of) blocks having a cloud point in the polymer.

In contrast to this, when a similar gel was prepared using the above-mentioned Pluronic F-127 in which polyethylene oxide was bonded to both ends of polypropylene oxide, the present inventors have found that the gel completely dissolved in water after standing for several hours.

From the viewpoint of suppressing the cytotoxicity during non-gelation to the lowest possible level, it is preferable to use a TGP that can be gelled at a concentration with respect to water, that is, (polymer)/(polymer + water) × 100 (%), of 20% or less (furthermore 15% or less, in particular 10% or less).

The molecular weight of the TGP used in the present invention is preferably 30,000 or more and 30 million or less, more preferably 100,000 or more and 10 million or less, and furthermore preferably 500,000 or more and 5 million or less.

In the method of the present invention, the step of culturing a cell population in a thermoreversible gelation polymer (TGP) may be carried out by inoculating the cell population separated from the epithelial tissue by mixing with the thermoreversible gelation polymer using a known method, and then incubating. Typically, the following method can be used: a cell population separated from epithelial tissue is separated into a TGP solution, the TGP is gelled at a temperature higher than the sol-gel transition temperature, and then a medium such as a culture solution is added for culturing.

Culturing in the present invention can be carried out under conditions normally used in the art. For example, typical culture conditions include culturing at 37°C and 5% CO₂. In addition, culturing can be performed at normal atmospheric pressure (atmospheric pressure). The culture period is not limited as long as the cell population in TGP does not become confluent, and for example, it is 12 hours or more, 24 hours, 2 days or more, 4 days or more, 8 days or more, 10 days or more, 12 days or more, 14 days or more, 16 days or more, 17 days or more, 18 days or more, 20 days or more, 22 days or more, 24 days or more, 36 days or more, 48 days or more; from the viewpoint of increasing the number of epithelial stem cells in the epithelial tissue, it is preferably 10 days or more, more preferably 12 days or more, and still more preferably 14 days or more.

Culturing can be carried out in containers of any size and shape. The medium for dissolving TGP and the medium for adding to gelled TGP are not particularly limited as long as they can maintain survival of cells, and typically, those containing amino acids, vitamins, and electrolytes as main components can be used. In the present invention, the medium for dissolving TGP and the medium for adding to gelled TGP may be common or different. In one embodiment of the present invention, the medium for dissolving TGP and the medium for adding to gelled TGP are based on a basal medium for cell culture. Such basal medium includes, but is not limited to, for example, DMEM (Dulbecco's Modified Eagle Medium), MEM (Minimum Essential Media), F12 (Ham's F-12 Nutrient Mixture), DME, RPMI (Roswell Park Memorial Institute) 1640, MCDB (MCDB102, 104, 107, 120, 131, 153, 199, etc.), L15, SkBM, RITC80-7, CnT-PR, etc. Many of these basal media are commercially available and their compositions are also known. The basal medium may be used as it is with a standard composition (for example, as it is on the market), or the composition may be appropriately changed depending on the cell type and cell conditions. Therefore, the basal medium used in the present invention is not limited to those having a known composition, and it includes those in which one or more components are added, removed, increased or decreased in their amounts.

In addition to the above, the medium may contain one or more additives such as sera, growth factors (for example, EGF, insulin, etc.), steroid preparation components, selenium components and the like. The sera may be heterologous sera or allogeneic sera. Allogeneic sera are preferred, and autologous sera are particularly preferred among allogeneic sera. The concentration of a serum is not particularly limited and the serum may be contained in a medium added to the TGP gel at 3%, 5%, 10% or 20%. It is preferably 10%. In one embodiment of the present invention, the medium for dissolving TGP and the medium for adding to gelled TGP consist of common components except that the medium for dissolving TGP does not contain serum.

The cell population obtained by the method of the present invention is more active than the cell population cultured by a method not comprising the step of culturing the cell population in a thermoreversible gelation polymer (hereinafter, may be referred to as control cell population 1). In one embodiment, the cell population obtained by the method of the present invention is more active than the cell population cultured by the same method of the present invention except that the cell population is separated from the epithelial tissue by a method other than the separation treatment in which Dispase I and trypsin are combined (hereinafter, may be referred to as control cell population 2). Examples of such activities include, but are not limited to, epithelial maintenance ability, tissue engraftment ability and epithelial tissue regeneration ability, etc. Here, higher activity means that, based on the activity of the control cell population 1 or 2, without limitation, the activity is for example 5% or more, 10% or more, 20% or more, 30% or more, 40% or more, 50% or more, 60% or more, 70% or more, 80% or more, 90% or more, or 100% or more higher.

In the present invention, the epithelial maintenance ability means that the tissue maintains the traits of a healthy epithelial tissue. In one embodiment of the present invention, maintenance of healthy epithelial tissue traits can be confirmed by the expression of a protein related to one or more indices selected from cytokeratin, epidermal growth factor receptors, epithelial cell adhesion factors and epithelial stem cell markers or a gene encoding said protein. Thus, in the cell population obtained by the method of the invention, compared to the control cell population 1 or 2, the expression of a protein related to one or more indices selected from cytokeratin, epidermal growth factor receptors, epithelial cell adhesion factors and epithelial stem cell markers or a gene encoding said protein is maintained or enhanced. Preferably, the expression of all proteins related to cytokeratin, epidermal growth factor receptors, epithelial cell adhesion factors and epithelial stem cell markers or genes encoding said proteins is maintained or enhanced.

In the present invention, the tissue engraftment ability means an engraftment ability of the cell population to the tissue to which it is applied. In one embodiment of the present invention, tissue engraftment ability is confirmed by, without limitation, the expression of a protein related to one or more indices selected from cytokeratin and epithelial cell adhesion factors or a gene encoding said protein. Therefore, in the cell population obtained by the method of the present invention, the expression of a protein related to one or more indices of cytokeratin and epithelial cell adhesion factors or a gene encoding said protein is maintained or enhanced, as compared to the control cell population 1 or 2.

In the present invention, the epithelial tissue regeneration ability means an epithelial tissue regeneration ability of the tissue to which the cell population is applied. In one embodiment of the present invention, epithelial tissue regeneration ability can be confirmed by, without limitation, expression of a protein related to an epithelial stem cell marker or a gene encoding said protein. Therefore, in the cell population obtained by the method of the present invention, the expression of a protein related to an epithelial stem cell marker or a gene encoding said protein is maintained or enhanced, as compared to the control cell population 1 or 2.

In one embodiment of the present invention, the protein related to cytokeratin may be, but is not limited to, one or more proteins selected from CK1, CK2, CK3, CK4, CK5, CK6, CK7 and CK8. The expression level of the protein can be confirmed using one or more monoclonal antibodies or polyclonal antibodies selected from, but are not limited to, anti-AE1/AE3 antibody, anti-34βE12 antibody, anti-CAM5.2 antibody, anti-D5/15B4 antibody, anti-OV-TL12/30 antibody, anti-E3 antibody, anti-DC10 antibody, anti-RCK108 antibody, anti-Ks20.8 antibody. Cytokeratin is preferably an anti-AE1/AE3 antibody.

The epidermal growth factor receptor may be EGFR. The expression level of said protein can be confirmed using, without limitation, an anti-EGFR antibody. The epithelial stem cell marker may be, but is not limited to, one or more markers selected from pax6, p63, E-cadherin, p75 and K14. It is preferably p63 and/or p75, and more preferably p63 and p75. These proteins can be confirmed using, without limitation, one or more monoclonal antibodies or polyclonal antibodies selected from anti-pax6 antibody, anti-p63 antibody, anti-E-cadherin antibody, anti-75 antibody, anti-K14 antibody.

The epithelial cell adhesion factor may be, but is not limited to, one or more proteins selected from integrins, cadherins, claudins, and nexons. The expression level of said protein can be confirmed using, without limitation, one or more monoclonal antibodies or polyclonal antibodies selected from anti-integrin antibody, anti-cadherin antibody, anti-claudin antibody, and anti-nexon antibody. It is preferably an anti-integrin antibody, and for example, an anti-integrin β antibody is preferable.

In one embodiment of the present invention, the epithelial maintenance ability is the maintenance or enhancement of expression of one or more proteins selected from the group consisting of cytokeratin, EGFR, integrin β, p63 and p75. In one embodiment of the present invention, the tissue engraftment ability is the maintenance or enhancement of expression of one or more proteins selected from the group consisting of cytokeratin and integrin β. In one embodiment of the present invention, the epithelial tissue regeneration ability is the maintenance or enhancement of expression of one or more proteins selected from the group consisting of p63 and p75.

In one embodiment of the present invention, the cell population obtained by the method of the present invention shows enhanced expression of one or more proteins selected from the group consisting of cytokeratin, EGFR, p63, p75 and integrin β or genes encoding said proteins, as compared to the control cell population 1 or 2. In another embodiment of the present invention, the cell population obtained by the method of the invention shows enhanced expression of all proteins selected from the group consisting of cytokeratin, EGFR, p63, p75 and integrin β or genes encoding said proteins, as compared to the control cell population 1 or 2. In the cell population obtained by the method of the present invention, based on the control cell population 1 or 2, the expression of one or more proteins selected from the group consisting of cytokeratin, EGFR, p63, p75 and integrin β or genes encoding said proteins is enhanced by, without limitation, for example 5% or more, 10% or more, 20% or more, 30% or more, 40% or more, 50% or more, 60% or more, 70% or more, 80% or more, 90% or more, or 100% or more.

Maintenance or enhancement of the expression of these proteins or genes encoding said proteins is either one or both of the following: the maintenance or enhancement of the expression of a protein or a gene encoding said protein in some or all of the cells constituting the cell population; or the increase in the number of cells positive for these indices (e.g., p63-positive cells, p75-positive cells) in the cell population.

Techniques for measuring the expression level of a protein or a gene encoding said protein described in detail above are well known in the art, and examples of the method for measuring the expression level of a protein include, but are not limited to, for example, Western blotting, EIA, ELISA, RIA, immunohistochemistry, immunocytochemistry, flow cytometer, mass spectrometry, etc. using the above-detailed monoclonal or polyclonal antibodies; and examples of the method for measuring gene expression levels include, but are not limited to, for example, Northern blotting method, Southern blotting method, DNA microarray analysis, RNase protection assay, PCR method such as RT-PCR and real-time PCR, and in-situ hybridization method, etc.

In one embodiment, in the method of the present invention all steps are performed in vitro. As detailed above, one embodiment of the present invention may include, but is not limited to, a step of shredding and/or separating the collected epithelial tissue, in particular the buccal mucosa. As detailed above, the method of the invention includes, but is not limited to, as detailed above, a step of separating the epithelial tissue. In another embodiment, the method of the present invention comprises a step performed in vivo, including, but not limited to, for example, a step of collecting an epithelial tissue, in particular buccal mucosa, from a subject. In one embodiment of the present invention, the epithelial tissue to be collected may be the buccal mucosa.

Another aspect of the present invention relates to a cell population produced by the method of the present invention. The cell population of the present invention is characterized by being more active than the control cell population 1 or 2. Details regarding the high activity are as described above. In one embodiment, the cell population of the present invention has an activity selected from the group consisting of epithelial maintenance ability, tissue engraftment ability and epithelial tissue regeneration ability, which is higher than that of the control cell population 1 or 2. Furthermore, in one embodiment, in the cell population of the present invention, the amount of expression of one or more proteins selected from the group consisting of cytokeratin, EGFR, p63, p75 and integrin β or genes encoding said proteins is higher than that in the control cell population 1 or 2. Since the cell population of the present invention has high activity, it exhibits effects superior to the control cell population 1 or 2 in various therapeutic applications. In particular, when the amount of expression of one or more proteins selected from the group consisting of cytokeratin, EGFR, p63, p75 and integrin β or genes encoding said proteins is high, upon application (transplantation) of the cell population to the tissue, the tissue engraftment ability and epithelial tissue regeneration ability continuously function for a long period of time, leading to the enhancement of the therapeutic effect.

### <Method of increasing proliferating ability of cells in epithelial tissue>

A method for enhancing the proliferating ability of cells in an epithelial tissue comprises a method comprising a step of culturing the epithelial tissue in a thermoreversible gelation polymer.

The "epithelial tissue" and "thermoreversible gelation polymer" in the present invention are as described in detail above.

The step of culturing the epithelial tissue in a thermoreversible gelation polymer in the present invention is, in other words, a step of culturing the tissue in which the epithelial structure is maintained. Therefore, in one embodiment of the present invention, the epithelial tissue is not subjected to enzymatic treatment. In one embodiment of the present invention, the epithelial tissue may be shredded or crushed to the extent that the structure of the epithelial tissue is maintained. Shredding treatment is preferable from the viewpoint of permeability of a culture solution into the epithelial tissue.

In the step of culturing the epithelial tissue in a thermoreversible gelation polymer in the method of the present invention, the epithelial tissue may be inoculated and cultured in the thermoreversible gelation polymer by a known method. Typically, the following method can be used: the epithelial tissue is inoculated by mixing it with a TGP solution at a low temperature, the TGP is gelled at a temperature higher than the sol-gel transition temperature, and then a medium such as a culture solution is added for culturing.

In one embodiment of the present invention, there are substantially no separated cells in the polymer immediately after the epithelial tissue is inoculated in the thermoreversible gelation polymer. In one embodiment of the present invention, the separated cells generated in the processes of collecting, shredding or crushing the epithelial tissue are less than 20%, less than 10%, less than 8%, less than 6%, less than 2% or less than 1% of the total cell count in the polymer. "Total cell count in the polymer" refers to the cell count of all cells present in the polymer, including cells in the epithelial tissue of the polymer and separated cells.

Culturing in the present invention can be carried out under the conditions normally used in the art. For example, typical culture conditions include culturing at 37°C and 5% CO₂. In addition, culturing can be performed at normal atmospheric pressure (atmospheric pressure). The culture period is not limited as long as the cells proliferated outside the epithelial tissue in the TGP do not become confluent, and it is 12 hours or more, 24 hours, 2 days or more, 4 days or more, 8 days or more, 10 days or more, 12 days or more, 14 days or more, 16 days or more, 17 days or more, 18 days or more, 20 days or more, 22 days or more, 24 days or more, 36 days or more, 48 days or more; from the viewpoint of increasing the number of epithelial stem cells in the epithelial tissue, it is preferably 8 days or more, more preferably 12 days or more, still more preferably 15 days or more, and particularly preferably 25 days or more.

"Proliferating ability of cells in epithelial tissue" refers to the proliferating ability of at least one type of cells constituting the epithelial tissue. The proliferating ability of cells in epithelial tissue may be, but is not limited to, the proliferating ability of epithelial cells and/or epithelial stem cells. In one embodiment, the proliferating ability of cells in epithelial tissue may be the proliferating ability of epithelial cells. Since epithelial cells are relatively abundant throughout the epithelial tissue, in this embodiment the proliferation of epithelial cells can be confirmed by an inverted microscope or the like as proliferation to the outside of the epithelial tissue. In another embodiment, the proliferating ability of cells in epithelial tissue may be the proliferating ability of epithelial stem cells. Since epithelial stem cells are sparsely distributed near the basal layer of epithelial tissue, when epithelial stem cells proliferate, they slowly start to proliferate inside the epithelial tissue. Therefore, in this embodiment, the epithelial stem cells proliferate only inside the epithelial tissue until a certain period of time, and only after a certain period of time, proliferation can be confirmed by an inverted microscope or the like as the proliferation outside the epithelial tissue. The proliferating ability of cells inside the epithelial tissue is also included in the proliferating ability of cells in the epithelial tissue. Furthermore, even when the total number of cells constituting the epithelial tissue is decreasing, if at least one type of the cells constituting the epithelial tissue is proliferating, the proliferating ability of the cells in the epithelial tissue can be said to be enhanced. For example, when epithelial stem cells proliferate while epithelial cells decrease in the epithelial tissue, the total number of cells constituting the epithelial tissue decreases until the time point when the proliferation of the epithelial stem cells exceeds the decrease of the epithelial cells; however, even in such a case, as long as the epithelial stem cells proliferate, it can be said that the proliferating ability of the cells in the epithelial tissue is enhanced.

The epithelial tissue obtained by the method of the present invention has higher proliferating ability than the epithelial tissue cultured by the method not comprising the step of culturing epithelial tissue in a thermoreversible gelation polymer (hereinafter, may be referred to as control epithelial tissue). Proliferating ability can be measured by methods known in the art, and the methods include, without limitation, a method of counting viable cells contained in epithelial tissue after culturing for a predetermined time, or a method of observing cells proliferating from epithelial tissue in a time lapse during culturing for a predetermined time. Here, the term "high proliferating ability" means that, without limitation, based on the proliferating ability of the control epithelial tissue, the proliferating ability is, for example, 5% or more, 10% or more, 20% or more, 30% or more, 40% or more, 50% or more, 60% or more, 70% or more, 80% or more, 90% or more, or 100% or more higher. In other words, it means that the amount of the epithelial tissue produced by the method of the present invention is, based on viable cells in the control epithelial tissue, for example, 5% or more, 10% or more, 20% or more, 30% or more, 40% or more, 50% or more, 60% or more, 70% or more, 80% or more, 90% or more, or 100% or more larger. In one embodiment of the present invention, the epithelial tissue produced by the method of the present invention contains more epithelial stem cells than the control epithelial tissue. Therefore, in one embodiment, the method of the present invention is a method for enhancing the proliferating ability of epithelial stem cells in the epithelial tissue. In one embodiment, it is a method for enhancing only the proliferating ability of epithelial stem cells among the cells constituting the epithelial tissue. In another expression, it is a method of selectively enhancing the proliferating ability of epithelial stem cells in the epithelial tissue.

As a method for counting epithelial stem cells, without limitation, markers usually known as epithelial stem cell markers such as p63, p75, OLFM4, CD133, LGR5 and LRIG1 can be used. It is preferably p63 or p75, and more preferably p75. Techniques for measuring the expression level of a protein or a gene encoding said protein are as detailed above. The increase in epithelial stem cells may be an increase in cells carrying an epithelial stem cell marker. Another expression may be, but is not limited to, an increase in p63- or p75-positive cells. In another embodiment, in epithelial tissues with limited cell types, cell morphology can be utilized to identify epithelial stem cells. In one embodiment of the present invention, for example, in a cell population consisting of two types: epithelial cells and epithelial stem cells, the epithelial cells have a polygonal tatami-like morphology, whereas the epithelial stem cells have a relatively round morphology; therefore, these may be discriminated and counted in terms of such forms.

In one embodiment, after a step of culturing the epithelial tissue, the present invention may provide a step of separating the epithelial tissue to obtain a cell population. The separation treatment of epithelial tissue is as detailed above. Since the cell population thus separated contains a large amount of epithelial stem cells, the cell population obtained by the method comprising said step has high tissue regenerative power.

In one embodiment, after a step of culturing the epithelial tissue, the present invention may comprise a step of separating the epithelial tissue to obtain a cell population, and a step of further culturing the cell population generated by the separation treatment in a thermoreversible gelation polymer. The cell population obtained by this method is rich in epithelial stem cells and has high epithelial maintenance ability, tissue engraftment ability and epithelial tissue regeneration ability.

In one embodiment, the present invention comprises a step of preserving the epithelial tissue in a thermoreversible gelation polymer prior to the step of culturing the tissue in the thermoreversible gelation polymer. In one embodiment, storage in the thermoreversible gelation polymer in the present invention may be maintaining the collected epithelial tissue in the thermoreversible gelation polymer at a temperature in which cells in the epithelial tissue do not substantially proliferate. The temperature at which cells do not substantially proliferate may be, but is not limited, 4°C to 30°C, 10 to 30°C, preferably 20°C to 30°C, and particularly preferably 20°C to 25°C. The storage time may be 30 minutes to 12 hours, 1 hour to 8 hours, 2 to 6 hours, and more preferably 3 to 5 hours.

The medium that dissolves a TGP during storage is as described in detail above. In one embodiment, all steps in the method of the present invention are performed in vitro. In one embodiment, all steps or part of the steps in the method of the present invention are performed in vivo. The method of the present invention comprises, but is not limited to, a step performed in vivo, for example, a step of collecting an epithelial tissue from a subject. In one embodiment of the present invention, the epithelial tissue to be collected may be the oral mucosa, and the buccal mucosa is particularly preferable.

### <Cell population or epithelial tissue obtained by the present invention>

The cell population or epithelial tissue obtained by the present invention is useful for treating various diseases related to abnormalities, in particular damages of epithelial tissue. Therefore, in one embodiment, the cell population or epithelial tissue obtained by the present invention is intended for use in the treatment of diseases related to abnormalities, in particular damages of epithelial tissue. Since the cell population or epithelial tissue obtained by the present invention has similar properties peculiar to constituent cells except that it has higher activity or proliferating ability than the conventional cell population or epithelial tissue, it can be applied to at least tissues and diseases that can be treated with conventional cell populations or epithelial tissues. Examples of the tissue to be treated include, but are not limited to, from the viewpoint of effectively exerting a repair effect, preferably an epithelial tissue and/or a connective tissue adjacent to the epithelial tissue, and a muscle tissue; as more preferable examples, it is effective against damages such as defect, burn, crush, and stenosis, etc. of epithelial tissue. In one embodiment of the present invention, the damaged site of epithelial tissue may be, but is not limited to, a lumen such as the esophagus, bile duct, urethra, ureter, fallopian tube, vaginal wall, and gastrointestinal tract. In one embodiment of the present invention, the epithelial tissue may be the skin, scalp, epidermis, cornea and conjunctival, external ear, external auditory canal and lips, oral cavity, mucous membrane, gingiva, tongue, tongue mucosa, upper respiratory tract, nasal cavity, sinus, nasal mucosa, vocal cord, throat, and genitals, lower respiratory tract, trachea, bronchi, bronchial trees, lungs, esophagus, gastric mucosa, mucous membrane of small intestine or large intestine. Specific diseases referred to as examples of epithelial tissue damage include, but are not limited to, urethral stricture, ureter stenosis, esophageal stenosis, and other gastrointestinal epithelial disorders (e.g., due to post-surgery, post-irradiation, acid/alkali burns, etc.), injury to the ocular surface (e.g., corneal epithelium) (due to physical trauma, chemical burns, other inflammatory infections, etc.), bile duct stenosis (due to pancreatitis, intestinal (small intestine) damage, cancers of bile duct and pancreas, etc.), damage to the vaginal wall (caused by trauma during childbirth, prolapse of the vaginal wall due to chronic constipation), fallopian tube stenosis (due to inflammatory disease in the pelvis (PID), pelvic surgery causing a damage to fallopian tube, uterine fibroid, endometriosis, etc.) and the like.

Another aspect of the present invention relates to a pharmaceutical composition comprising a cell population or epithelial tissue obtained by the present invention. The pharmaceutical composition of the present invention may comprise, in addition to the cell population or epithelial tissue obtained by the present invention, various additional components such as a pharmaceutically acceptable carrier and a component that enhances survival, engraftment and/or function of the cell population or epithelial tissue obtained by the present invention, and other active components that are useful for treating a target disease. Components that enhance the survival, engraftment and/or function of cell populations or epithelial tissues include, but are not limited to, thermoreversible gelation polymers, matrigels, fibrin gels and the like. Any known additional components can be used, and those skilled in the art are familiar with these additional components.

Another aspect of the present invention relates to a method of treating a disease associated with an abnormality in epithelial tissue in a subject, the method comprises administering an effective amount of a pharmaceutical composition comprising the cell population or epithelial tissue obtained by the present invention to a subject in need thereof. The tissues and diseases targeted by the treatment method of the present invention are as described above for the cell population or epithelial tissue obtained by the present invention. In addition, in the treatment method of the present invention, a component that enhances the survival, engraftment and/or function of the cell population or epithelial tissue obtained by the present invention and other active components useful for treating a target disease and the like are used in combination with the cell population, epithelial tissue or pharmaceutical composition obtained by the present invention.

The treatment method of the present invention may further comprise a step of producing the cell population or epithelial tissue obtained by the present invention according to the production method of the present invention. The treatment method of the present invention may further comprise a step of collecting an epithelial tissue from the subject prior to the step of producing the cell population or epithelial tissue obtained by the present invention. In one embodiment, the subject from which the epithelial tissue is collected is the same individual who receives the treatment of administering the cell population or epithelial tissue or pharmaceutical composition obtained according to the invention, that is, the administration is autologous transplantation. In another embodiment, the subject from which the epithelial tissue is collected is a separate individual of the same species as the subject who receives the treatment of administering the cell population or epithelial tissue or pharmaceutical composition obtained according to the invention; that is, the administration is allotransplantation.

In the present invention, the term "subject" means any individual organism, preferably an animal, more preferably a mammal, even more preferably a human. In the present invention, the subject may be healthy or suffer from some disease; however, when the treatment of epithelial tissue abnormalities, in particular damage-related diseases is intended, typically the subject means a subject who has such a disease or who is at risk of suffering from such a disease.

In addition, the term "treatment" shall include all types of medically acceptable prophylactic and/or therapeutic interventions aimed at the cure, temporary remission or prevention of a disease. For example, the term "treatment" encompasses medically acceptable interventions with variety of purposes, including delaying or stopping the progression of abnormalities and in particular damage-related diseases of epithelial tissues, retraction or disappearance of lesions, prevention of the onset or recurrence of the diseases, and the like.

In the present invention, the effective amount is, for example, an amount capable of suppressing the onset or recurrence of a disease, alleviating the symptoms, or delaying or stopping the progression, and preferably it is an amount capable of preventing the onset and recurrence of the disease, or curing the disease. In addition, an amount that does not cause an adverse effect exceeding the benefit of administration is preferable. Such an amount can be appropriately determined by, for example, a test in an experimental animal such as a mouse, rat, dog or pig, or a disease model animal; and such a test method is well known to those skilled in the art. In addition, the size of a tissue lesion to be treated can be an important index for determining the effective amount.

Examples of the administration method include a method of directly applying to the tissue, or a method of dispersing in a TGP and applying the TGP to the tissue; the administration may also be performed by injection. The frequency of administration is typically once per treatment, however, when the desired effect is not obtained, multiple administrations are possible.

### [Examples]

### [Production Example 1]

42.0 g of N-isopropylacrylamide and 4.0 g of n-butyl methacrylate were dissolved in 592 g of ethanol. An aqueous solution prepared by dissolving 11.5 g of polyethylene glycol dimethacrylate (PDE6000, manufactured by NOF Corporation) in 65.1 g of water was added thereto, and the mixture was heated to 70°C under a nitrogen stream. While maintaining 70°C under a nitrogen stream, 0.4 mL of N,N,N',N'-tetramethylethylenediamine (TEMED) and 4 mL of a 10% ammonium persulfate (APS) aqueous solution were added and stirred for 30 minutes. Furthermore, 0.4 mL of TEMED and 4 mL of 10% APS aqueous solution were added 4 times at intervals of 30 minutes to complete the polymerization reaction. The reaction solution was cooled to 5°C or lower, diluted with 5 L of cold distilled water at 5°C, and concentrated to 2 L at 5°C using an ultrafiltration membrane having a molecular weight cutoff of 100,000.

The concentrated solution was diluted by adding 4 L of cooled distilled water, and the above concentration by ultrafiltration was performed again. The above dilution and concentration by ultrafiltration were repeated 5 times to remove those having a molecular weight of 100,000 or less. Those not filtered by this ultrafiltration (remaining in the ultrafiltration membrane) were collected, freeze-dried, and 40 g of the hydrogel-forming polymer of the present invention having a molecular weight of 100,000 or more ("hydrogel-forming polymer"-6) were obtained. 1 g of the hydrogel-forming polymer of the present invention ("hydrogel-forming polymer"-6) obtained as described above was dissolved in 9 g of distilled water under ice cooling to obtain a 10 wt% aqueous solution. The storage elastic modulus of this aqueous solution was measured at an applied frequency of 1 Hz using a stress-controlled rheometer (AR500, manufactured by TA Instruments), and it was 43 Pa at 10°C, 680 Pa at 25°C, and 1310 Pa at 37°C. This temperature-dependent change in the storage elastic modulus was repeatedly and reversibly observed.

### [Example]

From 4 humans (56 years of age (#1075), 17 years of age (#1076), 32 years of age (#1077), 46 years of age (#1079), # indicates the sample number), each four samples of buccal mucosal tissue (3 mm³) were collected from the oral cavity of each human. The samples were treated with Dispase I (1000 PU/mL) at 37°C for 60 minutes, and stopped by adding DMEM (Thermo Fisher Cat No. 11965-084) containing 10% of autologous serum of each patient. Then, large pieces of tissue among the undigested tissues were finely shredded. The tissue was then treated with trypsin-EDTA (0.25% solution) for 15 minutes, and the cell population was collected by centrifugation. This cell population contains cells separated by digestive fluid and some unseparated pieces of the tissue.

### [Comparative Example 1]

### <Plate culture>

Cell populations were cultured in DMEM (hereinafter referred to as "culture solution") containing human recombinant insulin (5 µg/ml), human recombinant EGF (10 ng/ml), penicillin (100 U/ml)/streptomycin (100 µg/ml), gentamicin (50 µg/ml), amphotericin B (0.25 µg/ml) and 10% autoserum at 37°C in a 5% carbon dioxide incubator (ESPEC BNA-111). The culture solution was changed every week and culturing was performed for about 5 weeks.

### [Example 1]

### <TGP culture>

1 g of the TGP prepared in the production example was dissolved in 9 ml of the culture solution, and the cell population was inoculated in the TGP. After gelling the TGP at 30°C, the culture solution was added onto the TGP and cultured at 37°C in a 5% carbon dioxide incubator. The culture solution on the TGP was exchanged every week and culturing was performed for about 5 weeks.

### (1) Immunostaining

The plate culture sample of Comparative Example 1 and the TGP culture sample of Example 1 were collected at the 4th week from the culture, 4°C PBS was added to each sample, and the samples were washed by 3-times repetition of stirring and centrifugation. Then, the samples were fixed with formalin and hardened with paraffin to make a block of cell population, which was cut into 10 µm to produce a tissue section. After deparaffinization, the tissue sections were reacted with AE1/AE3 monoclonal antibody (Abcam, UK, Cat No. ab27988), anti-EGFR monoclonal antibody (Abcam, UK, Cat No. ab182618), anti-integrin β monoclonal antibody (Abcam, UK, Cat No. ab210515), anti-P63 monoclonal antibody (Abcam, Cat No. ab53039), and anti-p75 monoclonal antibody (Abcam, UK, Cat No. ab221212) diluted with PBS, at room temperature for 60 minutes. After washing 3 times with PBS, each tissue section was reacted with anti-mouse or anti-rabbit antibody (Goat Anti-Mouse IgG H&L (HRP) (ab205719), Goat Anti-Rabbit IgG H&L (HRP) (ab205718)) at room temperature for 60 minutes. Then, the tissue section was colored with iView DAB detection kit (Ventana Medical Systems Inc., USA).

### [Results of immunostaining]

As a result of staining with anti-AE1/AE3 antibody, in Comparative Example 1, about half of the cell population was negative for the anti-AE1/AE3 antibody, and cytokeratin was not considered to be expressed. Since these negative cells were not fibroblasts because of their shape, they were considered to be the dedifferentiated epithelial cells. In addition, the cells showing a positive result in Comparative Example 1 had low staining intensity. In contrast, all the cell populations of Example 1 showed strong positives.

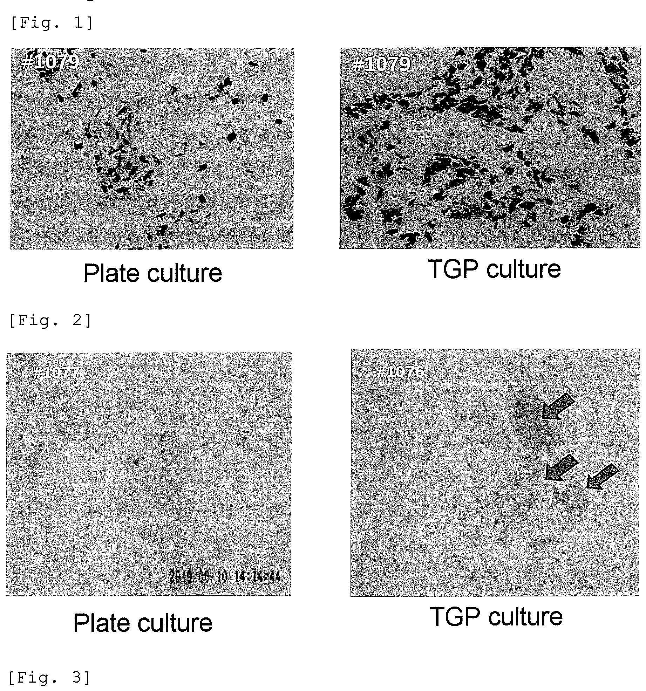

From this result, it was confirmed that when the cells were cultured in plate culture, the expression of cytokeratin was lost or decreased in a part of the cell population derived from epithelial tissue. On the other hand, when cultured in the TGP gel, it was found that the expression of cytokeratin was maintained or enhanced. Therefore, culturing in the TGP gel can maintain the traits of epithelial cells in the cell population. An example of the result is shown in Fig. 1 (observed at a magnification of 200 times).

As a result of staining with EGFR, the cell population of Comparative Example 1 was mostly negative. About half of the cell populations of Example 1 were EGFR positive. An example of the result is shown in Fig. 2. Arrows in the photograph of TGP culture indicate stained cells. From this, it was confirmed that when the cells were cultured in plate culture, the expression of EGFR was lost or decreased in a part of the cell population derived from epithelial tissue. On the other hand, when cultured in the TGP gel, it was found that the expression of EGFR was maintained or enhanced. Therefore, it was found that the TGP culture of the cell population separated from epithelial tissue can maintain the traits of epithelial cells. An example of the result is shown in Fig. 2 (observed at a magnification of 1000 times).

As a result of staining with integrin β, a part of the cell populations of both of Comparative Example 1 and Example 1 were positive, but compared with Comparative Example 1, there were more positive cells and the color development intensity was higher in the cell population of Example 1. An example of the result is shown in Fig. 3 (observed at a magnification of 500 times). Thus, it can be seen that the TGP culture of the cell population separated from epithelial tissue imparts engraftment ability to the cell population.

As a result of staining with p63, all the cell populations in Comparative Example 1 were negative; however, in Example 1, about half of the cell populations were positive. From this, it was found that p63-positive cells in the cell population derived from epithelial tissue disappeared in the plate culture, whereas p63-positive cells were maintained or increased in the TGP culture. Therefore, it was suggested that culturing in the TGP gel can increase epithelial stem cells in the population of cells.

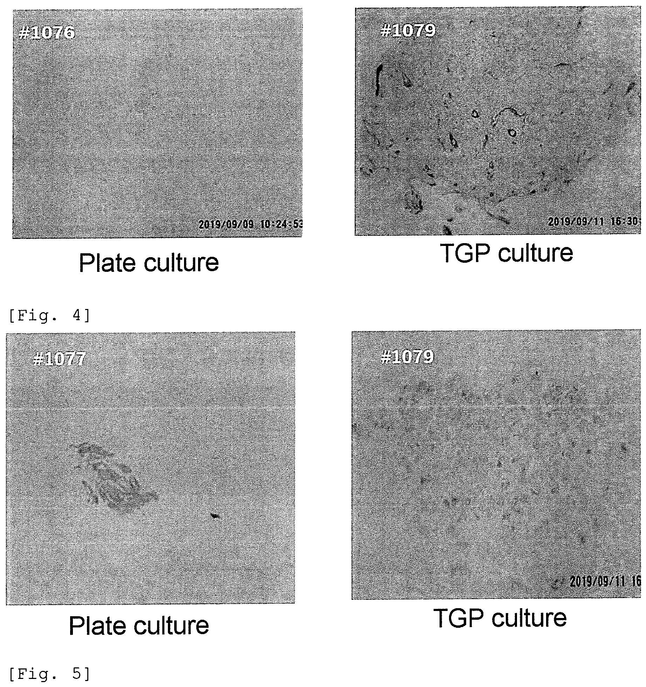

As a result of staining with p75, all the cell populations in Comparative Example 1 were negative; however, in Example 1, about half of the cell populations were positive. From this, it was found that p75-positive cells in the cell population derived from epithelial tissue disappeared in the plate culture, whereas p63-positive cells were maintained or increased in the TGP culture. Therefore, it was suggested that culturing in the TGP gel can increase epithelial stem cells in the population of cells. An example of the result is shown in Fig. 4 (observed at a magnification of 500 times).

### [Summary]

Table 1 shows a summary of the results of immunostaining with anti-AE1/AE3 antibody, anti-EFGR antibody, anti-integrin β antibody, anti-p63 antibody, and anti-p75 antibody. Table 2 shows meanings of the symbols representing the results in Table 1.

**[Table 1]**

| | AE1/AE3 | EGFR | Integrin β | p63 | p75 |
|---|---|---|---|---|---|
| TGP culture | ++++/S | +++/M | +/S | +++/M | +++/M |
| 2D culture | +++/W | +/W | +/W | - | - |

**[Table 2]**

| **Index-1** | Symbol |
|---|---|
| All of the cell population was stained | ++++ |
| 50% or more of the cell population was stained | +++ |
| 10% or more of the cell population was stained | ++ |
| Several percent of the cell population was stained | + |
| Not stained | - |

| **Index-2** | Symbol |
|---|---|
| Strong staining | S |
| Moderate staining | M |
| Weak staining | W |

### [Example 2]: Number of viable cells after transport and culture of epithelial tissue

From the oral cavity of humans (54 years of age (#1080), 21 years of age (#1081), 17 years of age (#1082), 36 years of age (#1083)), each 4 samples of oral mucosal tissue (3 mm³) were collected and washed by the same procedure as in Example 1. Each piece of tissue was cut evenly and placed in two 10-ml test tubes (samples A and B) respectively.

### [Transport of tissue]

Sample A: 1 g of the TGP prepared in the production example was dissolved in 9 ml of DMEM at 4°C to prepare a 10% TGP solution, 10 ml of this solution was added to the test tube A containing the tissue piece, and gelation was performed at 30°C for 1 hour; then, the test tube was maintained at about 20°C using a Bio Box (SUGIYAMA-GEN Co., Ltd., Cat No.SBE-10W) and Thermo Storage 20 (SUGIYAMA-GEN Co., Ltd., Cat No.TP-20-350), and transported for 4 hours.

Sample B: 10 ml of PBS was added to the test tube B containing the buccal tissue piece, and the test tube was transported at 4°C for 4 hours.

### [Measurement of number of viable cells]

Tissue pieces of samples A and B were washed with PBS at 4°C, 0.05 mg each of the tissue pieces was weighed, treated with trypsin-EDTA solution (0.25%) at 37°C for 30 minutes, and then digested with collagenase II solution (1 mg/ml) at 37°C for 2 hours. After filtering with a 100 µm cell strainer (PLS, Cat No: 43-50100-03), the samples were centrifuged (150 rpm, 5 minutes) and suspended in 2 ml PBS. 100 µl was fractionated from each sample, 400 µL of 0.4% trypan blue solution was added, and the cells were counted on a cytometer. The results are shown in Table 3.

**[Table 3]**

| Sample No. | Number of cells after transport (x10⁶) | |
|---|---|---|
| | Sample A | Sample B |
| #1080 | 0.87 | 0.45 |
| #1081 | 0.46 | 0.11 |
| #1082 | 0.32 | 0.18 |
| #1083 | 0.41 | 0.22 |

### [Results]

Since many viable cells were confirmed in all of the epithelial tissues transported in the TGP gel, it can be seen that storage in the TGP gel is suitable for transporting epithelial tissue.

### [Example 3]: Confirmation of proliferativeness by transport and culture of epithelial tissue

From the oral cavity of a human (34 years of age) (#1084), a part of the healthy buccal tissue piece was collected, and the sample was collected and transported by the same procedure as in Example 2 except that ECS (corning glucose solution (Euro-Collins), Product Number 99-408-CM) was used instead of PBS.

### [Confirmation of proliferativeness]

Each tissue piece of samples A and B was washed with 4°C PBS. Each tissue piece of samples A and B was inoculated in 10% TGP in which 1 g of the TGP prepared in the production example was dissolved in 9 ml of a culture solution. After gelling at 30°C for 1 hour, the culture solution was added and the samples were cultured in a 5% carbon dioxide gas incubator (sample A-TGP, sample B-TGP). The proliferativeness of the tissue during culturing was confirmed with an inverted microscope.

A part of the tissue pieces of samples A and B was added to a 25 cm² flask together with DMEM, and cultured (sample A-DMEM, sample B-DMEM). Furthermore, a part of the tissue piece of sample B was added to a 25 cm² flask containing 10 ml of Cnt-PR and cultured (sample B-Cnt-PR). Inverted microscope images of sample A-DMEM and sample A-TGP cultured for 7 days and 17 days are shown on the left side of Fig. 5. Images of sample B-DMEM, sample B-CnT-PR, and sample B-TGP are shown on the right side of Fig. 5. All have a magnification of 10 times except that sample A-TGP has a magnification of 40 times, and sample B-TGP at Day 17 has a magnification of 40 times.

When the tissue pieces of sample A were cultured in DMEM or TGP, cell proliferation was confirmed at a relatively early stage (sample A-DMEM, -TGP). As shown in Fig. 5, in sample A-DMEM, many cells were shown to proliferate from the tissue piece at day 17 of culturing. In sample A-TGP, cell proliferation was confirmed at the earliest time point among all samples. As shown in Fig. 5, in sample A-TGP, it can be seen that an extremely large number of cells are proliferating from the tissue piece at day 17 of culturing.

When the tissue pieces of sample B were cultured in DMEM, Cnt-PR or TGP (samples B-DMEM, -Cnt-PR, -TGP), the growth rates were all slow, but those cultured in the TGP was relatively fast. As shown in Fig. 5, in sample B-DMEM, while the cells were shown to slightly proliferate from the tissue piece, fibroblasts started to proliferate from the tissue piece at day 7 of culturing, and the bottom of the culture dish was covered with fibroblasts at day 17 of culturing. In sample B-Cnt-PR, cells slightly proliferated from the tissue piece at day 17 of culturing. In sample B-TGP, many cells proliferated from the tissue piece at day 17 of culturing. Since cell proliferation was excellent in sample A regardless of the culture medium, it was found that when TGP was used for transport, TGP caused less damage to the tissue compared to ECS.

Tissue pieces of sample A-TGP, sample A-DMEM, sample B-TGP and sample B-DMEM at day 21 of culturing were washed with 4°C PBS. Tissue pieces of the samples A and B were washed with PBS at 4°C, each treated with trypsin-EDTA solution (0.25%) at 37°C for 30 minutes, and then digested with collagenase II solution (1 mg/ml) at 37°C for 5 hours. After filtering with a 100 µm cell strainer, the samples were centrifuged (150 rpm, 5 minutes) and suspended in 2 ml PBS. 100 µl was fractionated from each sample, 400 µL of 0.4% trypan blue solution was added, and the cells were counted on a cytometer. The results are shown in Table 4. "E:N" in the table indicates the ratio of epithelial cells to non-epithelial cells.

**[Table 4]**

| Culture method | Number of cells after culturing (x10⁶) | |
|---|---|---|
| | Sample A | Sample B |
| TGP culture | 0.77 | 0.44 |
| | E:N = 28:72 | E:N = 43:57 |
| Plate culture | 0.45 | 0.12 |
| | E:N = 73:27 | E:N = 62:38 |

The highest number of cell was exhibited with the sample A (TGP) cultured in the TGP after transport.

The lowest number of cell was exhibited with the sample B (ECS) cultured in plate culture after transport. In addition, even in the sample B in which the number of viable cells after transport was low in Example 2, a sufficient number of viable cells was confirmed when subjected to the TGP culture. From this, it was found that the TGP enhances the proliferating ability of cells in epithelial tissue, and in particular, the TGP restores the proliferating ability of epithelial tissue in which the number of viable cells has decreased.

Furthermore, it was clarified that the proportion of non-epithelial cells became high in the samples subjected to TGP culture. While epithelial cells have a polygonal tatami-like morphology, non-epithelial cells have a round morphology that is characteristic of epithelial stem cells, and in Example 1, epithelial stem cell markers typified by p63 and p75 have increased; therefore, the proliferated non-epithelial cells were considered to be epithelial stem cells. Therefore, it was found that culturing an epithelial tissue in the TGP proliferates epithelial stem cells in the epithelial tissue.

### [Example 4]: Measurement of IGF-1 concentration in culture supernatant

Buccal mucosal tissue (3 mm³) was collected from the oral cavity of each of 5 humans (sample numbers: U7S1202 to U7S1206). It was treated with Dispase I (1000 PU/mL) at 37°C for 60 minutes, and stopped by adding DMEM (Thermo Fisher Cat No. 11965-084) containing 10% of each human's autologous serum. Then, large pieces of tissue among the undigested tissues were finely shredded. The tissue was then treated with trypsin-EDTA (0.25% solution) for 15 minutes and the cell population was collected by centrifugation. This cell population contains cells separated by digestive fluid and some unseparated pieces of the tissue.

The cell population obtained as described above is subjected to plate culture (2D) or TGP culture (3D), and the concentration of insulin-like growth factor 1 (IGF-1) in each culture supernatant was measured by ELISA (enzymelinked immunosorbent assay). Human IGF-I/IGF-1 Quantikine ELISA Kit (R&D systems, Cat# DG100B) was used for the measurement.

ELISA was performed using a quantitative sandwich enzyme immunoassay. A monoclonal antibody specific for IGF-1 was pre-coated on a microplate. Standard samples and pretreated samples were pipetted into wells such that the immobilized antibody was bound to IGF-1. After washing away unbound material, an enzyme-bound polyclonal antibody specific for IGF-1 was added to the wells. After washing to remove unbound antibody enzyme reagents, a substrate solution was added to the wells, and a color was developed in proportion to the amount of IGF-1 bound in the first step. Color development was stopped and color intensity was measured using a microplate reader.

The results are shown in Table 5.

**[Table 5]**

| Sample No. and culture method | IGF-1 concentration of culture supernatant (ng/ml) | Number of cells (x10⁴ cells) | Amount of IGF-1 per 10⁶ cells | 2D/3D value |
|---|---|---|---|---|
| U7S1206-2D | 2.3425 | 0.5 | 93.70 | 5.61 |
| U7S1206-3D | 2.0895 | 2.5 | 16.72 | |
| U7S1202-2D | 3.035 | 0.5 | 60.70 | 80.83 |
| U7S1202-3D | 1.99 | 26.5 | 0.75 | |
| U7S1203-2D | 3.299 | 0.5 | 65.98 | 175.04 |
| U7S1203-3D | 1.847 | 49 | 0.38 | |
| U7S1204-2D | 3.459 | 0.5 | 138.36 | 25.81 |
| U7S1204-3D | 2.1445 | 8 | 5.36 | |
| U7S1205-2D | 3.052 | 0.5 | 122.08 | 8.71 |
| U7S1205-3D | 2.8045 | 4 | 14.02 | |

As shown in Table 5, plate-cultured (2D) cells produced more IGF-1 than TGP-cultured (3D) cells.

### [Example 5]: Flow cytometry (FCM) analysis

Using the cell population obtained in Example 4, the expression of cell surface markers in cells cultured in plate culture (2D) or TGP culture (3D) was determined using anti-human Pan Cytokeratin Antibody Cocktail PE Conjugate [clone AE1 + AE3] (V2330PE) (NSJ Bioreagents, Japan) and APC anti-human CD140b (PDGFRβ) antibody (BioLegend, Japan). Flow cytometry was performed using BD^{™} LSR II flow cytometer. The data were analyzed using BD^{™} FACS DIVA Software.

Cells were collected from the culture, added to the tube, and centrifuged at 500 × g for 5 minutes. The supernatant was removed and the resulting product was incubated with 50 µl of human serum for 5 minutes at room temperature. The primary antibody mix was added to each tube, stirred vigorously and incubated in the dark at room temperature for 15 minutes. The supernatant was discarded and 200 µl of 1% paraformaldehyde was added to obtain a sample. Flow cytometry of the obtained sample was performed by BD^{™} LSR II flow cytometer. Cellular data were obtained using forward scatter and side scatter, and gating was used to exclude debris. The analysis was performed using BD^{™} FACS DIVA Software, wherein a gate for population was defined (% Gated), and mean fluorescence intensity (MFI) values were generated by the software based on the fluorescence intensity of the gated cell population.

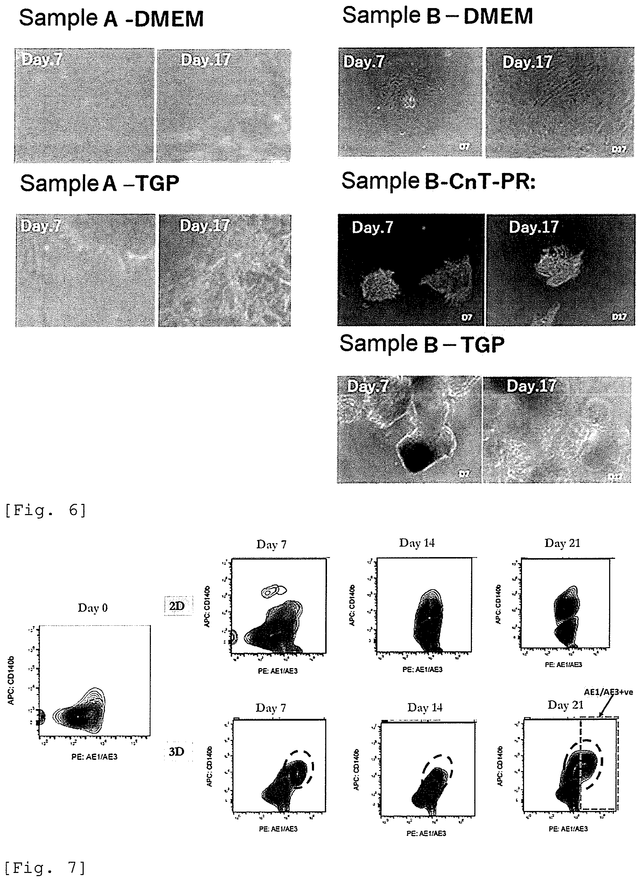

The results are shown in Figs. 6 and 7.

As shown in Fig. 6, while the cell population (day 0) freshly obtained from the tissue by enzymatic treatment forms one population, when comparing the case wherein plate culture is advanced (days 7 to 21 of 2D) and the case wherein TGP culture was advanced (days 7 to 21 of 3D), a difference was observed in the distribution of cells constituting the cell population. Morphologically, at day 7 or later of the plate culture, some fibroblasts were also observed; whereas in the TGP culture, epithelial cells remained as they were even at day 7 or later days.

Figure 7 shows the results of markers AE1, AE3, and CD140b superimposed on the results of typical flow cytometry of the cell population cultured in the plate culture (2D) and the cell population cultured in the TGP (3D). In the plate-cultured cell population, the number of cells showing positive for both of the epithelial cell markers AE1 and AE3 was extremely small at 1.12%. On the other hand, in the TGP-cultured cell population, 55.3% of the cells were positive for both of the epithelial cell markers AE1 and AE3.

From these results, it was shown that the cell surface of the cells obtained by enzymatic treatment from the tissue was damaged, and this damage was hardly recovered by the plate culture, but was recovered by the TGP culture.

### [Example 6]: Evaluation of CK3 by RT-PCR

Evaluation of CK3 (also referred to as cytokeratin 3 or KRT3) in the plate-cultured or TGP-cultured cells (at day 7 of culturing) was performed by RT-PCR.

The mRNA expression in the cell lysates of the plate culture and TGP culture was analyzed. Collected cells were stored in RNAlater^{™} Stabilization Solution (AM7020, Invitrogen) for further analysis. Total RNA was isolated using Nucleospin RNA XS kit (Macherey-Nagel). RT-PCR was performed using One Step TB Green PrimeScript PLUS RT-PCR Kit (Perfect Real Time) (Takara Bio Inc., Japan) and Thermal Cycler Dice^{®} Real Time System Lite (TP700, Takara Bio Inc.). qPCR was performed using mRNA qRT-PCR (Takara Bio Inc.) and Thermal Cycler Dice^{®} Real Time System Lite (TP700, Takara Bio Inc.).

A delta-delta Ct method was used to quantify mRNA. Such method is an approximation method that measures the relative level of mRNA between two samples by comparing with a second RNA used as a normalization standard. GADPH was used as the normalization standard.

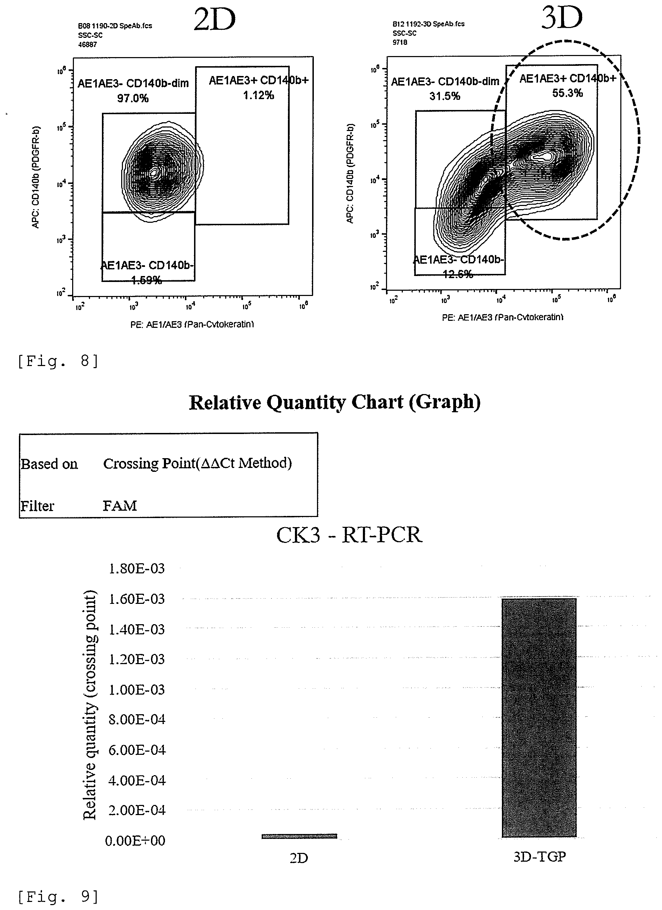

The results are shown in Table 6 and Fig. 8.

Here, the primers used are as follows.

### GAPDH:

Forward primer: GCACCGTCAAGGCTGAGAAC (SEQ ID NO: 1)
Reverse primer: TGGTGAAGACGCCAGTGGA (SEQ ID NO: 2)

### CK3:

Forward primer: GGGAACAGATCAAGACCCTCAAC (SEQ ID NO: 3)
Reverse primer: TGTGCCTGAGATGGAACTTGTG (SEQ ID NO: 4)

**[Table 6]**

| Target | | Sample | Relative quantification | SD+ | SD- |
|---|---|---|---|---|---|
| 1 | GAPDH | U7S1206-2D | 1.00E+00 | 1.02E+00 | 9.87E-01 |
| 1 | GAPDH | U7S1206-3D | 1.00E+00 | 1.04E+00 | 9.65E-01 |
| 2 | CK3 | U7S1206-2D | 3.61E-05 | 3.67E-05 | 3.56E-05 |
| 2 | CK3 | U7S1206-3D | 1.59E-03 | 3.63E-03 | 6.96E-04 |

As shown in Table 6 and Fig. 8, it was revealed that the TGP culture (3D) expressed more CK3 than the plate culture (2D).

### [Example 7]: Cell transplantation method (animal test)

Urethral stricture was generated in New Zealand white male rabbits by a coagulation method using a blunt tip coagulator for 30 seconds. Anterior and posterior urethrograms were obtained and endoscopic images were also recorded. After confirming the generation of an appropriate stricture, the rabbits were observed for 3-4 weeks and urethrography was performed to confirm the structure. At this time, a small amount of buccal tissue was also collected and cultured. Cells were prepared from the buccal tissue in the same manner as in Example 4 to obtain cells derived from the buccal tissue. The cells derived from buccal tissue were cultured in plate culture (2D) or TGP culture (3D) for 7 to 14 days, and used in the experiment.

The rabbits were subjected to urethral incision under general anesthesia before and after urethrography, and endoscopic images were also recorded. Then, the plate-cultured (2D) cells and/or the TGP-cultured (3D) cells were transplanted. Immediately after the endoscopic damage, one rabbit was sacrificed and the urethra was histopathologically examined. The rabbits receiving cell transplantation were sacrificed 6-12 days after urethography and endoscopy, and the urethra was collected for histopathological evaluation.

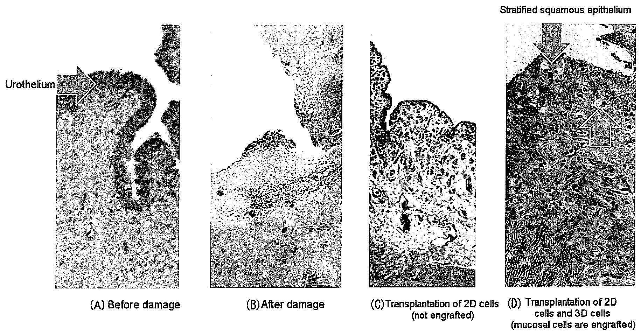

The results are shown in Fig. 9.

The distal urethra which has not been damaged by coagulation or not underwent urethral incision was maintained as a control showing the urothelium lining the lumen (Fig. 9A). Specimens collected immediately after the coagulation damage showed severe inflammation and lack of epithelial lining (Fig. 9B). No epithelial engraftment occurred when only the plate-cultured (2D) cells were used for transplantation (Fig. 9C) or only the TGP-cultured (3D) cells were used for transplantation (not shown). The rabbits transplanted with a mixture of the plate-cultured (2D) and TGP-cultured (3D) cells had an initial coagulation damage, and had engraftment of the buccal mucosal squamous epithelial lining at the site of urethral incision where there was a stenosis (Fig. 9D).

From this result, it is considered that the epithelial engraftment has been achieved by using a combination of the cell population in which its properties as epithelial cells were restored by the TGP culture, and the cell population that produces IGF-1 by the plate culture.

### [Industrial applicability]

The composition of the present invention comprises cells cultured in plate culture and cells cultured in a thermoreversible gelation polymer, and it can repair damages in various epithelial tissues. In particular, since the composition of the present invention has a cell population exhibiting the properties as shown in the above examples, the composition is able to repair various damaged epithelial tissues, including (i) repair of a corneal defect (eyeball surface, that is, corneal epithelium), (ii) cure of the skin ulcer, burn and wound, and skin regeneration, (iii) urethra reconstruction, (iv) ureter stenosis repair, (v) repair of esophageal mucosal damage, (vi) repair of nasolacrimal duct obstruction, (vii) repair of bile duct stenosis, (viii) repair of frontal sinus obstruction.

## Claims

1. A composition for repairing damage to an epithelial tissue, comprising:
(1) cells derived from the same or different epithelial tissue as the epithelial tissue to be repaired, which have been cultured in plate culture,
(2) cells derived from the same or different epithelial tissue as the epithelial tissue to be repaired, which have been cultured in a thermoreversible gelation polymer.

2. The composition according to claim 1, wherein the cells cultured in plate culture produce insulin-like growth factor 1 (IGF-1).

3. The composition according to claim 1 or 2, wherein the cells cultured in a thermoreversible gelation polymer are positive for epithelial cell markers AE1 and AE3.

4. The composition according to any one of claims 1 to 3, wherein the epithelial tissue to be repaired include epithelial tissues of oral cavity, nasal cavity, trachea, alveoli, vascular endothelium, lymphatic endothelium, bile duct, urinary tract, sweat gland, cornea, esophagus, stomach, mammary duct, endometrial membrane, cervix of uterus, large intestine, colon, kidney or bladder.

5. The composition according to any one of claims 1 to 4, wherein the cells cultured in plate culture and the cells cultured in a thermoreversible gelation polymer are cells derived from the same epithelial tissue.

6. The composition according to claim 5, wherein the cells cultured in plate culture and the cells cultured in a thermoreversible gelation polymer are derived from the oral mucosa.

7. The composition according to any one of claims 1 to 6, wherein the thermoreversible gelation polymer is composed by binding a plurality of blocks having a cloud point selected from the group consisting of polypropylene oxide, copolymers of propylene oxide and other alkylene oxides, poly N-substituted acrylamide derivatives, poly N-substituted methacrylamide derivatives, copolymers of N-substituted acrylamide derivatives and N-substituted methacrylamide derivatives, polyvinyl methyl ether, and partially acetylated polyvinyl alcohol, and a hydrophilic block.

8. The composition according to any one of claims 1 to 7, wherein the thermoreversible gelation polymer is composed by binding poly-N-isopropylacrylamide and polyethylene oxide.
